# EUROPEAN PATENT SPECIFICATION

(11) **EP 1 335 960 B1**
(45) Date of publication and mention of the grant of the patent: **05.08.2009**
(21) Application number: 01992750.8
(22) Date of filing: 22.10.2001
(51) Int. Cl.: C09K 3/10

(54) **SELF-SEALING MEDIA COMPRISING SUPER-ABSORBENT MATERIALS**
SELBSTDICHTENDE MEDIEN AUS SUPERABSORBERN
SUPPORTS AUTO-ETANCHE COMPRENANT DES MATERIAUX TRES ABSORBANTS

(30) Priority: 31.10.2000 US 699364
(43) Date of publication of application: 20.08.2003
(73) Proprietor: Porex Corporation, Fairburn, GA 30213-2828 (US)
(72) Inventor: YAO, Li, Peachtree City, GA 30269 (US); LI, Xingguo, Peachtree City, GA 30269 (US)
(74) Representative: MacDougall, Donald Carmichael
(86) International application number: PCT/US2001/047056
(87) International publication number: WO 2002/036708

(56) References cited:
- EP-A- 0 301 753
- US-A- 4 924 860
- US-A- 5 156 811
- US-A- 5 175 046
- DATABASE WPI Section Ch, Week 199024 Derwent Publications Ltd., London, GB; Class A18, AN 1990-181430 XP002205719 & JP 02 117801 A (SUMITOMO BAKELITE CO), 2 May 1990 (1990-05-02)

## Description

### 1. FIELD OF THE INVENTION

The invention relates to permeable self-sealing media that seal when exposed to water, methods of making such media, and devices made from or comprising such media.

### 2. BACKGROUND OF THE INVENTION

### 2.1. SELF-SEALING MEDIA

Self-sealing media are gas- or liquid-permeable materials that become less so when exposed to a particular substance. Typical self-sealing media prevent or inhibit the passage of gas or liquid when contacted with an aqueous liquid or vapor, and are of great utility in a variety of filtering and venting applications. One application is the venting of air from syringes. The use of a self-sealing vent in this case can allow the expulsion of air from a syringe while preventing the expulsion of its contents, which may be hazardous. Another application is the prevention of sample overflow in pipettes. Other potential applications of self-sealing media include, but are not limited to, ventilation of liquid storage and/or delivery systems such as intravenous drug delivery systems.

In order for a self-sealing medium to be useful in a wide range of applications, it must respond (*i.e.*, seal) quickly when exposed to water, cause little or no contamination of aqueous solutions with which it comes in contact, and be capable of withstanding high back-pressures (*e.g.*, greater than about 7 psi) before again allowing the passage of gas or liquid. If the medium is to be used in medical applications, it may also need to be biocompatible (*e.g.*, free of potentially toxic chemicals).

U.S. Patent No. 4,340,067 discloses a syringe having a bypass element that allegedly allows the expulsion of air, but prevents the expulsion of blood. The bypass element is made of a hydrophilic material that swells when exposed to water. Although the hydrophilic materials that are disclosed (*i.e.*, porous filter papers and copolymers of polyvinyl chloride (PVC) and acrylonitrile) do absorb water to some extent, they do so too slowly to be of much use in other applications. The use of PVC copolymers in many applications is also limited by the fact that they are made using free-radical processes, and consequently may contain trace amount of initiators, monomers, plasticizers, and other toxic molecules.

U.S. Patent Nos. 4,924,860, 5,125,415, 5,156,811, and 5,232,669 disclose self-sealing media that operate by a different mechanism. These media are made of a porous plastic impregnated with a hydrophilic material such as carboxyl methyl cellulose (CMC), which forms a viscous, amorphous mass when contacted with water. Unfortunately, because CMC and related materials dissolve in water, they have no structural integrity when wet, and will readily leach from media that contain them. The contamination of solutions that contact such media can also occur in the form of metal or other ions found in cellulose powders. For example, sodium CMC readily releases sodium ions into water.

The usefulness of media such as that disclosed by U.S. Patent No. 5,156,811 is further limited by the length of time it takes for cellulose powders to increase the viscosity of water to a point where sealing occurs. It is further limited by the fact that conventional self-sealing materials such as CMC will only affect the passage of water through pores that contain them. This means, for example, that the majority of the pores of CMC-based self sealing media must contain particles of CMC if sufficient sealing is to occur upon contact with water. Conventional self-sealing media therefore contain as much as 10 to 20 weight percent cellulose powder.

The high cellulose content of conventional self-sealing media causes-several problems. For example, it increase the probability that cellulose particles will fall out of media, and contaminate their surroundings. And because conventional self-sealing media must contain such large amounts of sealing material, they must contain a proportionally smaller amount of the plastic matrix that provides the media with structure. This can have an adverse effect on the mechanical strength of the media, especially when wet.

A third type of self-sealing medium is disclosed by U.S. Patent Nos. 4,769,026 and 5,364,595. This material is made of a porous, hydrophobic plastic that has a small average pore size. It can therefore be used to avoid severe contamination problems associated with cellulose-based self-sealing materials. However, it can withstand only moderate back-pressures before allowing the passage of water.

### 2.2. SUPER-ABSORBENT MATERIALS

Materials have been developed during the past ten years that rapidly swell when contacted with water, but do not dissolve in water. These materials, which are referred to herein as "super-absorbent materials," but which are also known as "superabsorptive polymers," can absorb large amounts of water and retain their structural integrity when wet. *See* Tomoko Ichikawa and Toshinari Nakajima, "Superabsortive Polymers," Concise Polymeric Materials Encyclopedia, 1523-1524 (Joseph C. Salamone, ed.; CRC; 1999).

A variety of super-absorbent materials are known to those skilled in the art. For example, U.S. Patent No. 5,998,032 describes super-absorbent materials and their use in feminine hygiene and medical articles. Other examples are.disclosed by U.S. patent No. 5,750,046, which describes a water-swellable, super-absorbent foam matrix; and by U.S. patent No. 5,175,046, which discloses a super-absorbent laminated structure. Additional examples of super-absorbent material include, but are not limited to, those disclosed by U.S. Patent Nos. 5,939,086; 5,836,929; 5,824,328; 5,797,347; 4,820,577; 4,724,114; and 4,443,515.

### 3. SUMMARY OF THE INVENTION

This invention relates to permeable media that seal when exposed to aqueous liquids, methods of making such media, and devices made from or comprising such media. In general, media of the invention comprise a porous plastic matrix and a super-absorbent material. The super-absorbent material can, for example, be in the form of particles within the pores of the plastic matrix, can be larger inclusions within the matrix, or can form a layer on a surface of the matrix.

A first embodiment of the invention encompasses a porous self-sealing medium which comprises: a sintered porous plastic matrix having an average pore size; and a plurality of inclusions within the porous plastic matrix, the inclusions made of a super-absorbent material; wherein the average size of the inclusions is equal to or greater than the average pore size.

In the medium of the invention, the porous plastic matrix is made of an organic polymer such as, but not limited to, acrylic polymers; polyolefins; polyesters; polyamides; poly(ether sulfone); polytetrafluoroethylene; polyvinyl chloride, polycarbonates; polyurethanes; and mixtures thereof. A particularly preferred plastic is polytheylene.

Preferred super-absorbent inclusions are made of a polymer selected from the group consisting of: hydrolyzed starch acrylonitrile graft copolymer; neutralized starch-acrylic acid graft copolymer; saponified acrylic acid ester-vinyl acetate copolymer; hydrolyzed acrylonitrile copolymer, acrylamide copolymer; modified cross-linked polyvinyl alcohol, neutralized self-crosslinking polyacrylic acid; crosslinked polyacrylate salts; neutralized crosslinked isbutylene-maleic anhydride copolymers; and salts and mixtures thereof. Particularly preferred super-absorbents are sodium polyacrylic acid and the sodium salt of poly(2-propenamide-co-2-propenoic acid).

A second embodiment of the invention encompasses a process of making a porous self-sealing medium which comprises: forming a mixture of plastic particles and super-absorbent particles having an average size; and sintering the mixture at a temperature and pressure sufficient to form a porous material having an average pore size approximately equal to or less than the average size of the super-absorbent particles.

In the method of the invention, the plastic particles are made of an organic polymer such as, but not limited to: acrylic polymers; polyolefins; polyesters; polyamides; poly(ether sulfone); polytetrafluoroethylene; polyvinyl chloride; polycarbonates; polyurethanes; and mixtures thereof A particularly preferred plastic is polyethylene.

Preferred super-absorbent particles are made of a polymer selected from the group consisting of: hydrolyzed starch acrylonitrile graft copolymer; neutralized starch-acrylic acid graft copolymer; saponified acrylic acid ester-vinyl acetate copolymer; hydrolyzed acrylonitrile copolymer; acrylamide copolymer; modified crass-linked polyvinyl alcohol; neutralized self-crosslinking polyacrylic acid; crosslinked polyacrylate salts; neutralized crosslinked isobutylene-maleic anhydride copolymers; and salts and mixtures thereof. Particularly preferred super-absorbents are sodium polyacrylic acid and the sodium salt of poly(2-propenamide-co-2-propenoic acid).

A third embodiment of the invention encompasses a pipette tip which comprises: a hollow tube open at opposite first and second ends; a center member disposed between said opposite first and second ends; and a means for attaching the first end of the hollow tube to a suction device; wherein said center member comprises the a porous self sealing medium of the invention. The invention further encompasses a pipette comprising such a pipette tip.

### 3.1. BRIEF DESCRIPTION OF THE DRAWINGS

Self-sealing media of the invention can be used in a wide variety of applications and can be incorporated into innumerable devices. Some of these applications and devices can be better understood with reference to the figures described below:
FIG. 1 illustrates a self-sealing medium of the invention that comprises super-absorbent inclusions, and the effect of those inclusions when contacted with water;
FIG. 2 illustrates the structure and behavior of a typical compatibilizer;
FIG. 3A illustrates a Pipette tip of the invention;
FIG. 3B illustrates a pipette of the invention;
FIG. 3C illustrates a top view of a pipette tip of the invention;
FIG. 3D illustrates a second pipette tip of the invention; and
FIG. 3E illustrates a third pipette tip of the invention.

### 4. DETAILED DESCRIPTION OF THE INVENTION

This invention relates to self-sealing media, which are permeable to gases or non-aqueous liquids but which become less permeable when exposed to aqueous liquids or vapors. Self-sealing media of the invention comprise a porous plastic matrix and a super-absorbent material, which rapidly swells as it absorbs water with which it comes into contact, but is not readily soluble in water. Many super-absorbent materials can absorb greater than about 100, 200, 500, or 1000 percent (*i.e.*, greater than about 100, greater than about 200, greater than about 500, or greater than about 1000 percent) of their weight in water while maintaining their structural integrity.

Because they are porous, self-sealing media of the invention allow the passage of gases when dry. However, when they are contacted with water, the super-absorbent material they contain swells to clog their pores, blocking and/or inhibiting the passage of gases through them.

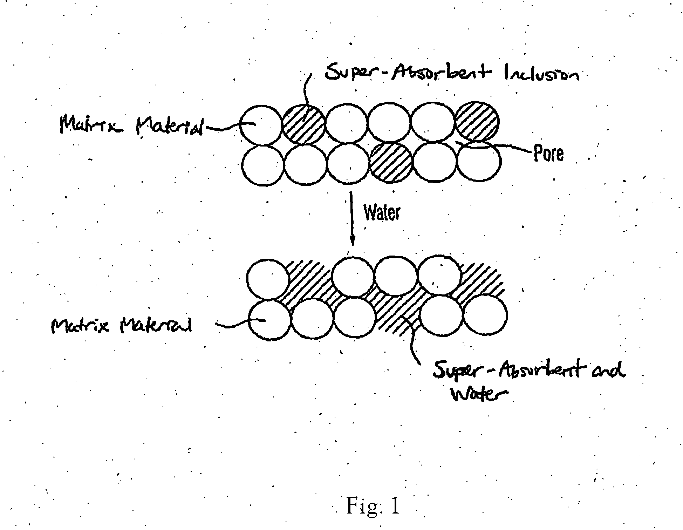

Super-absorbent materials are incorporated within porous matrices as inclusions. Inclusions are pieces of super-absorbent material that are about the same size or larger than the average pore size of the porous matrix in which they are trapped. Inclusions can therefore act in the same was as the non-porous portion of a porous matrix by defining its porous regions (*e.g.*, by forming part of a pore wall). FIG. 1 shows an example of a self-seating medium that comprises super-absorbent inclusions, and a representation of what happens to those inclusions when the medium is contacted with water.

An example of how compatibilizers work is shown in FIG. 4.

Self-sealing media of this invention can exhibit a number of desirable properties, including, but not limited to, short response times, little or no contamination of aqueous solutions with which they come into contact, the ability to withstand high back-pressures, and biocompatibility. Particular self-sealing media of the invention can withstand a water back-pressure of greater than about 7, 8, or 8.5 pounds per square-inch (psi). And the air flow rate of self-sealing media under an air pressure of 1.2 inches water can be greater than about 16,18, or 20 ml/minute. As discussed herein, the mechanical, physical, and chemical properties of self-sealing media can be adjusted by the appropriate selection of the porous matrix, super-absorbent, and optional compatibilizer. The process used to make the media can also affect their properties.

This invention is based, in part, on a discovery that super-absorbent materials can be incorporated into porous plastic matrices to provide self-sealing media. Unlike cellulose (*e.g.*, CMC) particles, which dissolve in water and can readily contaminate liquids that contact media containing them, super-absorbent materials absorb water while retaining their structural integrity. Consequently, media of this invention are less likely to contaminate liquids with which they come into contact.

It has also been found that, unlike cellulose-based sealing materials, many super-absorbent materials do not support microbial growth. This is shown in Example 2, below. Such growth can cause additional contamination problems that must be avoided in, for example, laboratory and medical applications.

Another advantage provided by media of the invention results from the rapidity and efficiency with which super-absorbent media can swell when contacted with water. This quality allows the manufacture of self-scaling media that contain relatively little super-absorbent material.

The ability of self-sealing media of this invention to function with much smaller amounts of seating material than used in prior self-sealing media offers several advantages. Media of the invention can have greater airflow than those which have been used in the past. Pipette tips made of media of the invention can therefore help increase the accuracy of the pipettes to which they are attached. The required use of only small amounts of super-absorbent materials can reduce the cost associated with the manufacture of self-sealing media that contain them. And, because media of the invention need not contain large amounts of sealing material, they can be stronger and more durable than prior self-sealing media.

### 4.1. MATERIALS FROM WHICH POROUS MATRICES CAN BE MADE

Self-sealing media of the invention comprise a porous matrix and inclusions of super-absorbent material. The porous matrix can be made of any material known to those skilled in the art, but it is preferably made of a maternal that is not soluble in the liquids with which it will come into contact (*e.g.*, water). Preferably, the material is inexpensive and can be used to provide a porous matrix of desired strength, durability, and porosity.

Examples of suitable materials include, but not limited to: metals, metal oxides, and alloys; ceramics; and inorganic and organic materials such as graphite, glass, paper, and organic and organometallic polymers; and mixtures thereof. Examples of metals, metal oxides, and alloys include, but are not limited to, Group IIIB, IVB, VB, VIB, VIIB, VIII, IB, IIB, IIIA, and IVA metals and oxides and alloys thereof. Specific metals include, but are not limited to, aluminum, titanium, chromium, nickel, copper, zinc, molybdenum, palladium, silver, copper, zinc, tungsten, platinum, and gold. Examples of alloys include, but are not limited to, stainless steel. Examples of ceramics include, but are not limited to, silica carbide, clays, and oxides of magnesium. Examples of papers include, but are not limited to, woven and non-woven cotton fiber based, glass fiber based, cellulose based, and carbon fiber based.

Preferred porous matrices are made of organic polymers. Examples of organic polymers include, but are not limited to: atactic and syntactic homopolymers; statistical, random, and alternating copolymers; periodic, block, and graft copolymers; regular and irregular single-stranded and double-stranded polymers; and mixtures thereof. Examples of specific organic polymers include, but are not limited to, polyolefins, olefin compolymer rubber, ethylene-vinyl acetate copolymer, metallocene-catalyzed polyolefin copolymer, and mixtures thereof. Polyolefins include, but are not limited to, polymers of ethylene, propylene, 1-butene, butadiene, 1-pentene, 1-hexene, 1-octene, isoprene, 2-methyl-i-butene, 3-methyl-i-butene, 4-methyl-i-pentene, and mixtures thereof. Specific polyolefins include, but not limited to: polyethylene and polypropylene; polyesters; polyamides such as nylon; poly(ether sulfone); polytetrafluoroethylene; polyvinyl chloride; polycarbonates; and polyurethanes.

Porous matrices can be made of amorphous olefin copolymer rubbers and random elastic copolymers that comprise a crosslinked olefin as a major component. Examples of olefin copolymer rubbers include, but are not limited to: olefin copolymer rubbers lacking a diene component, such as ethylene-propylene copolymer rubber, ethylene-butene-1 copolymer rubber, and propylene-ethylene copolymer rubber; ethylene-propylene nonconjugated diene copolymer rubbers such as ethylene-propylene-cyclopentadiene copolymer rubber, ethylene-propylene-4-hexadiene copolymer rubber, ethylene-propylene-cyclooctadiene copolymer rubber, ethylene-propylene-methylenenorbornene copolymer rubber, and ethylene-propylene-ethylidenenorbornene copolymer rubber; ethylene-butadiene copolymer rubber; and mixtures thereof.

Porous matrices can further be made of metallocene catalyzed polyolefins, examples of which include, but are not limited to, low density polyethylene, linear low density polyethylene, medium density polyethylene, high density polyethylene, polypropylene, ethylene-propylene rubber, ethylene-propylene-diene terpolymer, ethylene-vinyl acetate copolymer, ethylene-maleic anhydride copolymer, ethylene-ethyl acetate copolymer, and mixtures thereof.

As described elsewhere herein, a preferred method of making porous matrices is by sintering plastic particles. In a specific embodiment of the invention, these particles are substantially spherical and free of rough edges, and can thus be efficiently packed into a mold. Substantially spherical particles, and in particular those with smooth edges, also tend to sinter evenly over a well defined temperature range to provide a final product with desirable mechanical properties and porosity.

Known techniques can be used to fabricate spherical particles, including underwater pelletizing and hot melt blowing. Both techniques are commercially available and suitable for the processing of low melting temperature and high melt flow index polymeric materials.

Although typically limited to the production of particles having diameters of greater than about 36 µM, underwater pelletizing offers several advantages. First, it provides accurate control over the average size of the particles produced, in many cases thereby eliminating the need for an additional screening step and reducing the amount of wasted material. A second advantage of underwater pelletizing is that it allows significant control over the particles' shape.

Underwater pelletizing is described, for example, in U.S. Patent Nos. 6,030,558; 5,679,380; 5,623,012; 5,599,562; 5,593,702; 5,435,713; and 4,822,546, Thermoplastic particle formation using an underwater pelletizing typically requires an extruder or melt pump, an underwater pelletizer, and a drier. The thermoplastic resin is fed into an extruder or a melt pump and heated until semi-molten. The semi-molten material is then forced through a die. As the material emerges from the die, at least one rotating blade cuts it into pieces referred to herein as "pre-particles." The rate of extrusion and the speed of the rotating blade(s) determine the shape of the particles formed from the pre-particles, while the diameter of the die holes determine their average size. Water, or some other liquid or gas capable of increasing the rate at which the pre-particles cool, flows over the cutting blade(s) and through the cutting chamber. This coagulates the cut material (*i.e.*, the pre-particles) into particles, which are then separated from the coolant (*e.g.*, water), dried, and expelled into a holding container.

The average size of particles produced by underwater pelletizing can be accurately controlled and can range from about 0.014" (35.6 µM) to about 0.125" (318 µM) in diameter, depending upon the porous matrix. Average particle size can be adjusted simply by changing dies, with larger pore dies yielding proportionally larger particles. The average shape of the particles can be optimized by manipulating the extrusion rate and the temperature of the water used in the process.

### 4.2. SUPER-ABSORBENT MATERIALS

Each self-sealing medium of the invention comprises a super-absorbent material, which rapidly expands as it absorbs water. Super-absorbent materials are typically classified as starch graft copolymers or modified hydrophilic polyacrylates, and are distinguished from hydrogels by the substantially greater rate at which they absorb water. Specific examples of super-absorbent materials include, but are not limited to, hydrolyzed starch acrylonitrile graft copolymer; neutralized starch-acrylic acid graft copolymer; saponified acrylic acid ester-vinyl acetate copolymer; hydrolyzed acrylonitrile copolymer; acrylamide copolymer; modified cross-linked polyvinyl alcohol; neutralized self crosslinking polyacrylic acid; crosslinked polyacrylate salts; neutralized crosslinked isobutylene-maleic anhydride copolymers; and salts and mixtures thereof. Particularly preferred super-absorbents are sodium polyacrylic acid and the sodium salt of poly(2-propenamide-co-2-propenoic acid).

Specific super-absorbent materials include, but are not limited to, those disclosed by U.S. Patent Nos. 5,998,032; 5,939,086; 5,836,929; 5,824,328; 5,797,347; 5,750,585; 5,175,046; 4,820,577; 4,724,114; and 4,443,515. Examples of commercially available super-absorbent materials include, but are not limited to, AP80HS, sold by Stockhousen, Tuscaloosa, AL, and HySorb^{®} P7200, sold by BASF, Budd Lake, NJ.

It will be apparent to those skilled in the art that the selection of super-absorbent material(s) for use in a self-sealing medium of the invention will depend on a variety of factors, including the physical and chemical properties of the material. For example, if the super-absorbent material is to be incorporated or trapped within a porous matrix during the sintering of plastic particles, the super-absorbent material should be capable of withstanding the sintering conditions without significant degradation (*e.g.*, burning). Other factors to be considered when selecting a super-absorbent material include, but are not limited to, the amount of water it can absorb, its rate of water absorption, how much it expands when it absorbs water, its solubility in non-aqueous solvents with which it may come into contact, its thermal stability, and its biocompatibility.

The physical and chemical properties of a super-absorbent material depend, at least in part, on the physical and chemical properties of the specific molecules from which it is made. For example, the bulk properties of a super-absorbent material made from a particular polymer can depend on the average molecular weight and hydrophilicity of that polymer. The bulk properties of the super-absorbent material can further depend on the amount and type of crosslinking that holds the polymers together.

Crosslinking can be of at least two types, and mixtures thereof. A first type is covalent crosslinking, wherein polymers are covalently attached to one another by methods well known in the art. A second type is physical crosslinking, wherein polymers are associated by hydrogen bonding, ionic bonding, or other non-covalent interactions, which can provide crystalline or semi-crystalline super-absorbent materials. Super-absorbent materials that are covalently crosslinked are typically more durable than physically crosslinked materials, but often contain chemical residues from the crosslinking process. Consequently, chemically crosslinked super-absorbent materials may not be suitable for use in applications wherein the leaching of such residues must be avoided.

The durability and toughness of super-absorbent materials typically increase with increased crosslinking. However, the ability of super-absorbent materials to rapidly expand and absorb water can decrease with increased covalent crosslinking. For example, sodium polyacrylate-based super-absorbent materials contain long, interwoven polymer chains having a number of ionic functional groups. When contacted with water, the ionic functional groups disassociate to provide an ionized polymer network. Swelling of the material occurs as ionic crosslinking is eliminated, and is accelerated due to repulsions between anions bound to the polymeric chain. As the material swells, large void volumes are created, which can accommodate the absorption of water until the polymer matrix can no longer expand. The scale of expansion is determined, at least in part, by the degree of crosslinking. Without intermolecular crosslinking, super-absorbent materials would expand infinitely; *i*.*e*., they would dissolve.

The degree to which a super-absorbent material absorbs water is related to the concentration of ionic functional groups and crosslinking density in it. In general, water absorption increases with an increased concentration of ionic functional groups and/or a decrease in crosslinking density. Of course, when particles or inclusions of super-absorbent material are trapped within the porous matrix of a self-sealing material, their expansion is also restricted by the matrix surrounding them.

### 4.3. COMPATIBILIZERS

Super-absorbent materials are incorporated into the porous matrix of a super-absorbent self-sealing medium as inclusions.

The adhesion of super-absorbent particles to plastics such as polyethylene often does not occur naturally. However, compounds exist and can be made that will associate with the exterior and/or interior (*i.e*., pore wall) surfaces of porous matrices as well as with the surfaces of super-absorbent materials. These compounds are referred to herein as "interfacial compatibilizers," or simply as "compatibilizers."

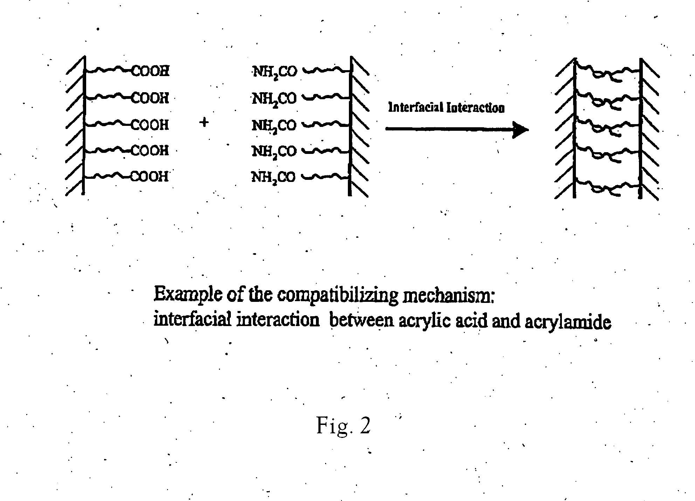

As shown in FIG. 2, compatibilizers comprise two segments, one of which is compatible with the exterior and/or interior surface of a porous matrix, and the other of which is compatible with the surface of a super-absorbent particle or inclusion. The two segments need not be different, but typically are. As used herein to describe a relationship between two materials, the term "compatible" means that contact between the two materials is thermodynamically favorable. Compatible materials can therefore interact with one another via covalent bonding, ionic bonding, hydrogen bonding, Van de Waals interactions, or other interactions that result in the materials' adhesion to one another.

As described elsewhere herein, compatiblizers can be incorporated into self-sealing materials of the invention during the sintering of mixtures that contain plastic particles (from which the porous matrix is formed) and super-absorbent particles. Preferred compatibilizers are also provided as very fine particles (*e.g*., having an average diameter of from about 10 to about 30 microns), and have an average size less than that of the average sizes of the plastic and super-absorbent particles. This diminishes the amount of compatibilizer necessary to adhere super-absorbent particles or inclusions to the surrounding porous matrix. Preferred compatibilizer particles are also substantially spherical so that they will readily flow into molds, and will melt in an even and controllable fashion. Such particles can be formed by techniques such as underwater pelletizing and hot melt blowing, as discussed above in Section 4.1.

Examples of compatibilizers include, but are not limited to, polyethylene-co-acrylic acid, polyethylene-g-acrylic acid, polyethylene-co-maleic anhydride, polyethylene-g-maleio anhydride; polyethylene-co-vinyl alcohol; polyethylene-co-glycidyl methacrylate; polyethylene-g-glycidyl methacrylate; and mixtures thereof.

The precise way in which a compatibilizer functions to provide a self-sealing medium of the invention will depend upon its structure and the chemical compositions of other materials within the medium. By way of example, and without being limited by theory, it is believed that the polyethylene segment of compatibilizers such as polyethylene-co-acrylic acid can readily associate with the polyethylene surfaces of certain porous matrices, while the compatibilizer's acrylic acid (or maleic anhydride) segment can associate with surfaces of super-absorbent inclusions that are highly polar.

### 4.4. PREPARATION OF SELF-SEALING MEDIA

As discussed elsewhere herein, self-sealing media of the invention comprise a porous matrix, which contains one or more channels through which gas or liquid molecules can pass. Porous matrices can be made by any method known to those skilled in the art including, but not limited to: sintering; the use of blowing agents and/or leaching agents; microcell formation methods such as those disclosed by U.S. Patent Nos. 4,473,665 and 5,160,674; drilling, including laser drilling; and reverse phase precipitation. Depending on how it is made, a porous matrix can thus contain regular arrangements of channels of random or well-defined diameters and/or randomly situated pores of varying shapes and sizes. Pore sizes are typically referred to in terms of their average diameters, even though the pores themselves are not necessarily spherical.

The average sizes, shapes, and number of pores in a material are typically determined by taking a cross-section of the material. Holes and depressions in the cross-section are considered pores. And while only two-dimensional sizes and shapes of those pores can be determined from the cross-section, information about their third dimension (*e.g*., their depth) can be determined from a second cross-section, orthogonal to the first.

The particular method used to form the pores or channels of a porous matrix and the resulting porosity (*i.e*., average pore size and pore density) of the porous matrix can vary according to the desired application for which the final self-sealing medium will be used. For example, small diameter pores of channels are preferred in cases where rapid self-sealing is desired and/or high back pressures are anticipated, while larger diameter pores or channels may be preferred in cases where small pressure gradients across the self-sealing medium are desired prior to sealing. The desired porosity of the matrix can also be affected by the matrix material itself, as porosity can affect in different ways the physical properties (*e*.*g*., tensile strength and durability) of different materials.

A preferred porous matrix of this invention has an average pore size of from about 1 µm to about 200 µm, more preferably from about 15 µm to about 100 µm, and most preferably from about 20 µm to about 50 µm. Mean pore size and pore density can be determined using, for example, a mercury porisometer, scanning electron microscopy, or atomic force microscopy.

In the process of preparing self-sealing media, a mixture is formed that comprises matrix material (*e*.*g*., particles of a plastic), super-absorbent material (*e*.*g*., particles of a super-absorbent), and optional compatibilizer material (*e.g*., particles of a compatibilizer). The materials are preferably in powder form, and are mixed to ensure an even distribution of each throughout the mixture. The mixture is then heated to the sintering temperature of the matrix material, optionally under pressure, to provide a self-sealing medium.

The super-absorbent and matrix materials are selected to ensure that the super-absorbent material will not bum or otherwise decompose at the sintering temperature of the matrix material. As discussed elsewhere herein, suitable matrix materials include, but are not limited to, polyethylene, polypropylene, polyester, nylon, poly(ether sulfone), polytetrafluoroethylene, polyvinyl chloride, polycarbonate, and polyurethane. Preferred matrix materials are polyolefins (*e*.*g*., polyethylene and polypropylene), and particularly preferred support materials are polyolefins that melt at about 120°C.

Preferably, the mixture comprises super-absorbent material in an amount of from about 1 to about 30 weight percent, more preferably from about 2 to about 15 weight percent, and most preferably from, about 3 to about 5 weight percent.

If an optional compatibilizer is used to adhere inclusions of super-absorbent material to interior and/or exterior surfaces of the porous matrix, it is added to the mixture in an amount that depends, at least in part, on its particle size and density. Typical mixtures comprise compatibilizer material in an amount of from about 0.1 to about 10 weight percent, preferably from about 0.2 to about 5 weight percent, and more preferably from about 0.5 to about 3 weight percent.

Those skilled in the art will recognize that the average pore size of the self-sealing medium will depend, at least in part, on the average particle size of the matrix material, the sintering temperature, and the pressure -if any- applied to the mixture during sintering. If the particles of super-absorbent material are smaller than the average pore size of the matrix, they will be trapped within pores of the matrix during the sintering process, and will be adhered to the walls of those pores if the mixture contains an optional compatibilizer. If particles of the super-absorbent material are larger than the average pore size of the matrix, they will be incorporated within the matrix as inclusions.

Sintering can occur on a belt or within a mold to yield a final product that can be cut into pieces of desired shape. The use of molds is preferred where the desired shape of the self-sealing medium is complex.

This first process (*i.e*., the sintering of a mixture of matrix, super-absorbent, and optional compatibilizer materials) offers several advantages. One advantage is economy, as the super-absorbent material is incorporated within the porous matrix as the matrix is formed. This process can also provide a relatively uniform distribution of super-absorbent particles or inclusions throughout the matrix.

Although the particular matrix used in this method to prepare a self-sealing medium will depend on a variety of factors, preferred porous matrices are made of high density polyethylene having a mean pore size of from about 15 µm to about 50 µm. Examples include, but are not limited to, part nos. X-6837, P-6516, and P-5973, available from Porex Technologies Corp., Fairburn, GA.

### 5. SELF-SEALING DEVICES

The self-sealing media of this invention can be incorporated into innumerable and varied devices. These include, but are not limited to, containers, pipette tips, intravenous, liquid delivery systems, and syringe caps. Other potential uses for, and devices comprising, the self-sealing media disclosed herein include, but are not limited to, the protection of tranducers, ink pen vents, the protection of vacuum pumps and/or systems, the protection of pneumatic components, use in the high speed filling of containers such as those used for batteries and beverages, emergency spill valves for chemical containers such as drums and bottles as well as those used on trains and other vehicles, "burp" or "blow-out" valves, use in the filling of refrigerant, brake, or hydraulic systems, and vents in items such as ink-jet cartridges and disk drives.

Additional uses of the self-sealing media of the invention will be apparent upon consideration of the following examples.

### 6. EXAMPLES

### 6.1. EXAMPLE 1: PREPARATION OF SELF-SEALING MEDIA

A self-sealing medium of the invention comprising the super-absorbent material sold under the name Water Lock G564 by Grain Processing Corporation, Muscatine, IA, and the compatibilizer sold under the name Flow Bead EA-209 by Sumitomo Seika, Osaka, Japan, was prepared as follows.

A mixture was formed by thoroughly mixing polyethylene R44 (94.5 weight percent), Water Lock G564 (5 weight percent), and Flow Bead EA-209 (0.5 weight percent). The mixture was then sintered at 10.3 bar (150 psi) for 3 minutes to provide a self-sealing material of the invention.

Because the melting temperature of Flow Bead EA-209 is much lower than that of polyethylene R44, it is believed to coat the surface of the polyethylene particles prior to and during the sintering process, instead of clogging the pores of the resulting material.

### 6.2. EXAMPLE 2: CONTAMINATION

The leaching of contaminants from self-sealing media that come into contact with aqueous solutions is a problem typical of prior self-sealing media. For example, media that contain sodium CMC can contaminate solutions with which they come into contact with both sodium ions and CMC.

Preferred self-sealing materials of the invention contaminate solutions with which they come into contact to a much smaller degree than do prior self-sealing materials. This was demonstrated by measuring, in part, the concentration of sodium leached from pipette tips using ICP-MS. Results of these measurements are provided below in Table 1.

**Table 1. Contamination Caused by Pipette Tips Made of Self-sealing Media of the Invention and of Conventional CMC-based Materials**

| Extraction | Material | [Na⁺] (2 sec.) | [Total Leaching] (2 sec.) |
|---|---|---|---|
| tip wall | 5% super-absorbent | 0 | 0 |
| tip wall | 20% CMC | 730 ppb | 4.260 ppm |
| plug & wall | 5% super-absorbent | 370 ppb | 14.8 ppm |
| plug & wall | 20% CMC | 1200 ppb | 24.0 ppm |

As shown elsewhere herein, pipette tips a self-sealing plug member disposed in a hollow tube. In Table 1, pipette tips are referred to by the self-sealing component used in the manufacture of their plug members. Pipette tips of this invention were prepared by sintering a mixture of 97 weight percent R44 polyethylene and 3 weight percent of the super-absorbent material Water Lock G564. These pipette tip were compared against commercially available pipette tips, which contain plug members made with approximately 20 weight percent CMC.

All of the pipette tips tested were of the same volume (200 µl). Total leaching refers to the concentration of matter leached from the pipette tip wall, or the porous plug and pipette tip wall.

### 6.3. EXAMPLE 3: BIOLOGICAL GROWTH

A microbial growth study was conducted to determine how self-sealing materials of the invention compared against commercially available pipette plug members.

Five pipette plugs of this invention, prepared as described in Example 1 above, and five taken from MBP pipette tips were aseptically removed from their packaging container and placed in a sterile 15 mL test tube. For initial colony counts of microbes contained on the insert, 5 mL of PBS with 0.1% Tween 80 were added to each tube. The tubes were shaken for 20 minutes at 36°C. Colony forming units (cfu) were obtained by aseptically spread plating 0.3 mL of filter eluants on tryptic soy agar (TSA) and counting the plate after a three day storage at 25 °C.

For evaluating the effect of 1 week open air storage at room temperature, the above process was repeated. However the inserts were stored in open 15 mL test tubes in the dark at 25°C and 95% humidity for 1 week. The inserts were then eluted and spread plated as mentioned. The results of these tests are shown below in Table 2.

**Table 2: Colony Forming Units (Cfu) of Pipette Tip Inserts Before and After One Week Exposure to Ambient Room Conditions**

| **Sample** | **cfu/mL** | |
|---|---|---|
| | **A** | **B** |
| 1 day | <3 | <3 |
| 2 days | 15 | <3 |
| 3 days | 3 | 3 |
| 4 days | 12 | 6 |
| 5 days | 24 | 9 |
| 1 week | 222 | 33 |
| 2 week | 144 | 166 |
| 3 week | 225 | 69 |
| 4 week | 276 | 96 |
| 5 week | 133 | 72 |

As shown in Table 2, the pipette plugs of this invention (identified as "A") initially contained less microbes than commercially available inserts (identified as "B"), which use approximately 20 weight percent CMC as a self-sealing material. Even after the one week storage, the Porex inserts contained significantly less microbes than the MBP inserts. This implies that the Porex inserts either suppressed microbial growth or did not support microbial proliferation as did the MBP inserts.

### 6.4. EXAMPLE 4: FILTER

Self-sealing media of the invention can be used in a variety of applications. In one application, a medium of the invention is used to provide an air filter that can be used in applications where contamination is not acceptable.

This filter comprises three layers. The first layer is made of 100 percent ultra-high molecular weight polyethylene (UHMWPE). On top of the first layer is a second layer, which is made of a blend of super-absorbent material (about 1 to about 20 weight percent), UHMWPE, and an optional compatibilizer. The third layer, which sits atop the second, is made of 100 percent UHMWPE. The filter can be made by layering three levels of powder in a mold, and sintering the levels together. Alternatively, the filter can be made by first sintering two sheets of 100% UHMWPE and a second sheet of the super-absorbent/UHMWPE blend. The sheets can be fused together by heating, optionally under pressure.

### 6.5. EXAMPLE 5: SELF-SEALING PIPETTE TIPS

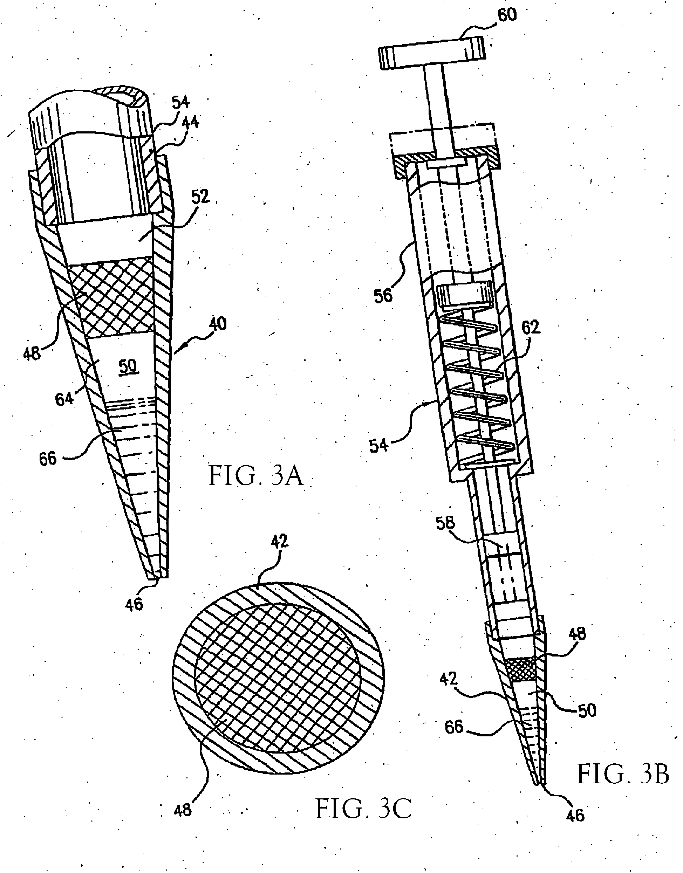

FIGS. 3A to 3E illustrate pipette and pipette tips of the invention. FIGS. 3A and 3B illustrate a pipette tip 40 for drawing and dispensing liquid samples. The pipette tip 40 basically comprises a tapering, hollow tubular member 42 of non-reactive material such as glass, open at its opposite first 44 and second 46 ends and a plug member 48 of the self-sealing medium of the invention disposed in the tubular member 42 to define a liquid sample chamber 50 between the plug member 48 and second end 46 of the tube. The plug member is also spaced from the first end 44 of the tube to define an air barrier or chamber 52 between the plug member and end 44 of the tube.

The first end 44 of the tubular member 42 is releasably secured to a suitable suction device 54 in a manner known in the field, as generally illustrated in FIG. 3B. Any suitable suction device for drawing a predetermined volume of liquid into the chamber 50 can be used, such as the volumetric pipettor illustrated in the drawings, or a suction pump, elastic bulb, bellows, or the like as are commonly used to draw liquids in the laboratory analysis field. The suction device 54 illustrated by way of example in FIG. 3B comprises a cylinder or a tube 56 and a piston 58 slidable in tube 56 and attached to a plunger 60 extending out of one end of tube 56 The opposite end of the tube 56 is secured to the first end 44 of the pipette tip 40. Piston 58 is urged upwardly to draw a predetermined volume of liquid equivalent to the piston displacement via return spring 62.

The plug member 48 is preferably force or pressure fitted securely into tube 42, under a sufficient pressure (*e*.*g*., about 1800 lb/in²) so that it is securely held and frictionally sealed against the inner wall of tube 42 although not physically attached to the inner wall by any adhesive or other extraneous material. The plug member has a tapering, frusto-conical shape of dimensions matching that of the tube 42 at a predetermined location intermediate its ends, so that the plug member will be compressed as it is forced into the tube and released at the desired position to seal against the inner wall of tho tube and define, a liquid sample chamber 50 of predetermined dimensions. The liquid sample chamber is arranged to be of predetermined volume greater than the liquid sample volume which will be drawn by one full stroke of the suction device. The dimensions of the chamber 50 beneath plug member 48 are such that there will be a substantial air gap 64 between plug member 48 and a drawn liquid sample 66 to reduce the risk of liquid actually contacting the plug member. The air gap is preferably in the range of from about 10 to about 40 percent of the total volume of chamber 50. Thus, one complete stroke of the suction device will draw only enough liquid to fill from about 60 to about 90 percent of the volume of chamber 50, as indicated in FIG. 3A.

FIG. 3C is a top view of the pipette tip 40. The plug member 48 is formed of a self-scaling medium of the invention. A particularly suitable material of the invention comprises hydrophilic polyurethane adhered to pore walls of porous a polyolefin.

In order to draw a liquid sample into pipette tube 54, the suction device or plunger is first depressed or compressed, as appropriate, and the tip end 46 is submerged below the surface of a liquid to be sampled. Any aerosol droplets drawn up into plug member 48 will come into contact with super-absorbent material adhered within pores of the plug member. The super-absorbent material in those pores will absorb the liquid and swell to eventually block them. Other pores in plug member 48 will still remain unblocked, however, and allow passage of gas through the plug member 48 to draw in and subsequently eject or blow out the sample. As long as the tubular member 42 is held more or less erect and not tilted or bounced during the sampling process, no liquid will come into contact with plug member 48 because the air gap 52 produced by the predetermined volume of sample chamber 50 is substantially greater than the volume of fluid drawn by one stroke of the suction device. When the sample has been drawn, the pipette and attached pipette tip are transferred carefully to a location above a vessel or sample collector into which the liquid sample is to be ejected for subsequent research or analysis. The sample is held in the tube under suction during this transfer procedure. Once the pipette tip is positioned above the collector, the suction device is actuated to blow gas or air back through the plug member and force the liquid sample out of the pipette.

If for some reason the liquid sample 66 actually contacts the plug member during the sampling procedure, sufficient liquid will be absorbed by the self-sealing medium to completely seal the plug member 48 to further passage of gas. Because the self-sealing medium does not contaminate the sample, however, the sample need not be discarded. This is of particular importance when samples contain, for example, material that is extremely expensive or difficult to isolate.

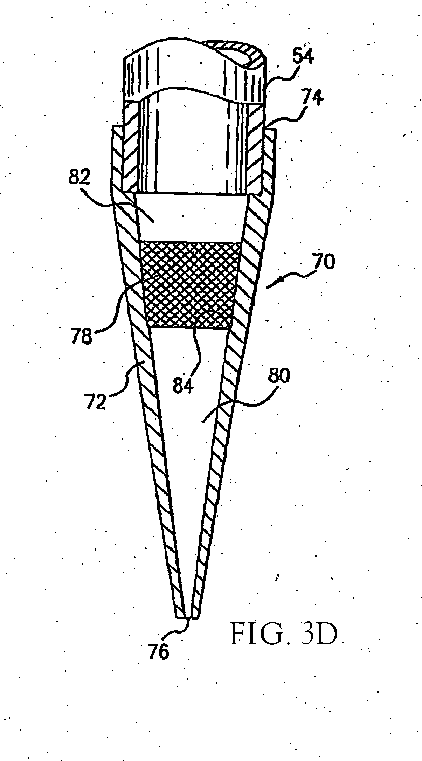

FIG. 3D illustrates a modified pipette tip 70 which again comprises a hollow, frusto-conical or tapering tubular member 72 for securing to a suitable pipette or suction device 54 at one end 74 so as to draw a liquid sample into the pipette through the opposite end 76. A plug member 78 which is of the same material as plug member 48 in the embodiment of FIGS. 3A to 3C is force or friction fitted into the member 72 at an intermediate point between its end so as to define a liquid sample chamber 80 on one side and an air barrier chamber 82 on the opposite side of plug member 78. However, in FIG. 3D the inner wall of member 72 is provided with a step or Shoulder 84 against which the plug member 78 is seated and which prevents movement of the plug member any further along the bore of tubular member 72. As in the previous embodiment, the sample chamber 80 has a volume substantially greater than that of a liquid sample drawn by one full stroke of the suction device, so that an air gap will be left between a drawn sample and the plug member. The modified pipette tip 70 operates in the same way as the pipette tip 40 of FIGS. 3A to 3C as described above.

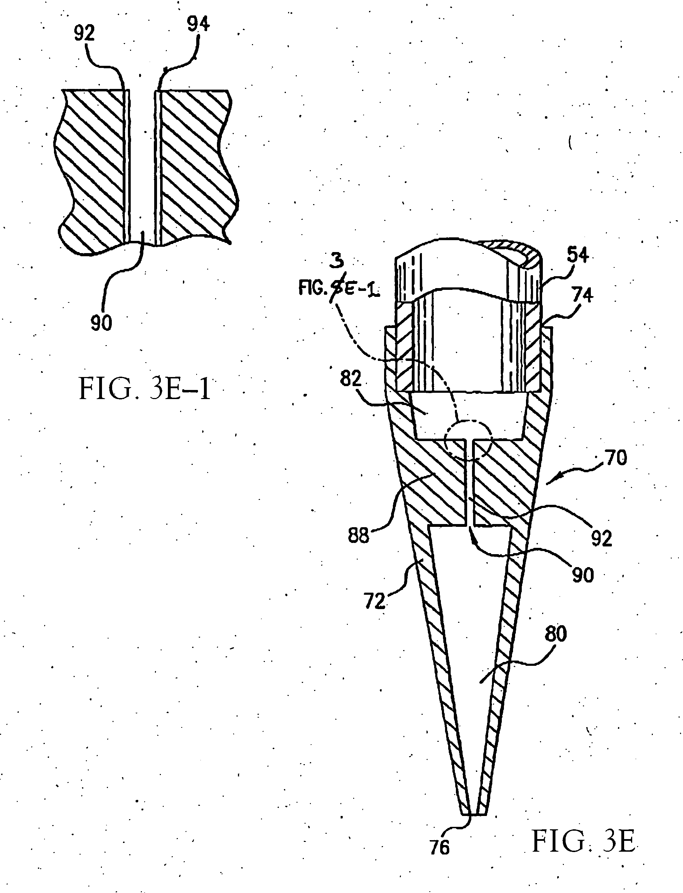

FIG. 3E illustrates a pipette tip of the invention which does not comprise a plug member, but instead consists of a center member 88 disposed between a liquid sample chamber 80 and an optional air barrier 82. The center member 88, which can be any shape and can be flat, curved or tapered at either end, contains at least one pore or channel 90 that allows air to flow from the liquid sample chamber80 to the optional air barrier 82. The inner wall 92 of the at least one pore or channel 90 is coated partially or entirely with a super-absorbent material 94. Consequently, the center member, which is simply a part of the pipette tip tubular member 72, acts as a plug member. When an aqueous solution enters the at least one pore or channel 90, the super-absorbent material 94 swells, thereby closing the at least one pore or channel 90 and preventing contamination of the suction device (*e*.*g*., pipette) to which the pipette tip is attached.

Pipette tips of this invention will greatly reduce the risk of contamination of the pipettor or suction device and resultant cross-over contamination to subsequent samples, and will also substantially reduce the risk to personnel when handling potentially infectious or other hazardous materials. Further, unlike other pipette devices, the self-sealing medium of the invention provides that when a sample does come into contact with the plug member, the sample is not contaminated by, for example, cellulose powder.

## Claims

1. A porous self-sealing medium which comprise:
a sintered porous plastic matrix having an average pore size; and
a plurality of inclusions within the porous plastic matrix, the inclusions made
of a super-absorbent material;
wherein the average size of the inclusions is equal to or greater than the average pore size.

2. The porous self-sealing medium of claim 1, wherein the sintered porous plastic matrix is made of an organic polymer selected from the group consisting of: acrylic polymers; polyolefins; polyesters; polyamides; poly(ether sulfone); polytetrafluoroethylene; polyvinyl chloride; polycarbonates; polyurethanes; and mixtures thereof.

3. The porous self-seallng medium of claim 2, wherein the sintered porous plastic matrix is made of polyethylene.

4. The porous self-sealing medium of claim 1, wherein the super-absorbent material is a polymer selected from the group consisting of: hydrolyzed starch acrylonitrile graft copolymer; neutralized starch-acrylic acid graft copolymer; saponified acrylic acid ester-vinyl acetate copolymer; hydrolyzed acrylonitrile copolymer; acrylamide copolymer; modified cross-linked polyvinyl alcohol; neutralized self-crosslinking polyacrylic acid; crosslinked polyacrylate salts; neutralized crosslinked isobutylene-maleic anhydride copolymers; poly(2-propenamide-co-2-propenoic acid); and mixtures and salts thereof.

5. The porous self-sealing medium of claim 4, wherein the super-absorbent material is sodium polyacrylic acid or the sodium salt of poly(2-propenamide-co-2-propenoic acid).

6. The porous self-sealing medium of claim 1, wherein the plurality of inclusions are present in an amount from 1 weight percent to 30 weight percent of the matrix.

7. The porous self-sealing medium of claim 1, wherein the plurality of inclusions are present in an amount from 3 weight percent to 5 weight percent of the matrix.

8. The porous self-sealing medium of claim 1, wherein the self-sealing medium can withstand a water back-pressure of greater than about 0.48 bar (7 psi).

9. The porous self-sealing medium of claim 1, wherein the average pore size of the porous plastic matrix ranges from 15 µm to 100 µm.

10. A process of making a porous self-sealing medium which comprises:
forming a mixture of:
plastic particles; and
super-absorbent particles having an average size; and
sintering the mixture at a temperature and pressure sufficient to form a porous material having an average pore size approximately equal to or less than the average size of the super-absorbent particles.

11. The process of claim 10, wherein the plastic particles are made of a polymer selected from the group consisting of: acrylic polymers; polyolefins; polyesters; polyamides; poly(ether-sulfone); polytetrafluoroethylene; polyvinyl chloride; polycarbonates; polyurethanes; and mixtures thereof.

12. The process of claim 10, wherein the plastic particles are made of polyethylene

13. The process of claim 10, wherein the super-absorbent particles are made of a polymer selected from the group consisting of: hydrolyzed starch acrylonitrile graft copolymer; neutralized starch-acrylic acid graft copolymer; saponified acrylic acid ester-vinyl acetate copolymer; hydrolyzed acrylonitrile copolymer; acrylamide copolymer; modified cross-linked polyvinyl alcohol; neutralized self-crosslinking polyacrylic acid; crosslinked polyacrylate salts; neutralized crosslinked isobutylene-maleic anhydride copolymers; poly(2-propenamide-co-2-propenoic acid); and mixtures and salts thereof.

14. The process of claim 13, wherein the super-absorbent particles are made of sodium polyacrylic acid or the sodium salt of poly(2-propenamide-co-2-propenoic acid).

15. The process of claim 10, wherein the mixture comprises from 3 weight percent to 5 weight percent super-absorbent particles.

16. A pipette tip which comprises:
a hollow tube open at opposite first and second ends;
a center member disposed between said opposite first and second ends; and
a means for attaching the first end of the hollow tube to a suction device; wherein said center member comprises the porous self-sealing medium of claim 1.

17. A pipette comprising the pipette tip of claim 16.

## Revendications

1. Milieu poreux atuto-étanche qui comprend:
une matrice plastique poreuse frittée ayant une taille moyenne de pore; et une pluralité d'inclusions à l'intérieur de la matrice plastique poreuse, les inclusions étant faites d'un matériau super-absorbant;
dans lequel la taille moyenne des inclusions est égale ou supérieure à la taille moyenne de pore.

2. Milieu poreux auto-étanche selon la revendication 1, dans lequel la matrice plastique poreuse frittée est faite d'un polymérie organique choisi dans le groupe constitué par: les polymères acryliques; les polyoléfines; les polyesters; les polyamides; une poty(éther sulfone); un polytétrafluoroéthylène; un polychlorure de vinyle; les polycarbonates; les polyuréthanes; et les mélanges de ceux-ci.

3. Milieu poreux auto-étanche selon la revendication 2, dans lequel la matrice plastique poreuse frittée est faite d'un polyéthylène.

4. Milieu poreux auto-étanche selon la revendication 1, dans lequel le matériau super-absorbant est un polymère choisi dans le groupe constitué par: un copolymère amidon hydrolysé acrylonitrile greffé; un copolymère amidon neutralisé-acide acrylique greffé; un copolymère ester d'acide acrylique saponifié-acétate de vinyle; un copolymère d'acrylonitrile hydrolysé; un copolymère d'acrylamide; un alcool polyvinylique réticulé modifié; un acide polyacrylique auto-réticulant neutralisé; les sels poyacrylates réticulés; les copolymères isobutylène-anhydride maléique réticulés neutralisés; un poly(acide 2-propénamide-co-2-propénoïque); et les mélanges et sels de ceux-ci.

5. Milieu poreux auto-étanche selon la revendication 4, dans lequel le matériau super-absorbant est un acide polyacrylique sodique ou le sel de sodium de poly(acide 2-propénamide-co-2-propénoïque).

6. Milieu poreux auto-étanche selon la revendication 1, dans lequel la pluralité d'inclusions sont présentes en une quantité de 1 pour cent en poids à 30 pour cent en poids de la matrice.

7. Milieu poreux auto-étanche selon la revendication 1, dans lequel la pluralité d'inclusions sont présentes en une quantité de 3 pour cent en poids à 5 pour cent en poids de la matrice.

8. Milieu poreux auto-étanche selon la revendication 1, dans lequel le milieu auto-étanche peut résister à une contre-pression d'eau supérieure à environ 7psi (0,48 bar)

9. Milieu poreux auto-étanche selon la revendication 1, dans lequel la taille moyenne de pore de la matrice plastique poreuse va de 15 µm à 100 µm.

10. Procédé de fabrication d'un milieu poreux auto-étanche, qui comprend:
la formation d'un mélange de:
particules plastiques; et de
particules super-absorbantes ayant une taille moyenne; et le frittage du mélange à une température et une pression suffisantes pour former un matériau poreux ayant une taille moyenne de pore approximativement égale ou intérieure à la taille moyenne des particules super-absorbantes.

11. Procédé selon la revendication 10, dans lequel les particules plastiques sont faites d'un polymère choisi dans le groupe constitué par: les polymères acryliques; les polyoléfines; les polyesters; les polyamides; une poly(éther-sulfone); un polytétrafluoro-méthylène; un polychlorure de vinyle; les polycarbonates; les polyuréthanes; et les mélanges de ceux-ci.

12. Procédé selon la revendication 10, dans lequel les particules plastiques sont faites d'un polyéthylène.

13. Procédé selon la revendication 10, dans lequel les particules super-absorbantes sont faites d'un polymère choisi dans le groupe constitué par: un copolymère amidon hydrolysé acrylonitrile greffé; un copolymère amidon neutralisé-acide acrylique greffé; un copolymère ester d'acide acrylique saponifié-acétate de vinyle; un copolymère d'acrylonitrile hydrolysé; un copolymère d'acrylamide; un alcool polyvinylique réticulé modifié; un acide polyacrylique auto-réticulant neutralisé; les sels polyacrylates réticulés; les copolymères isobatylène-anhydride maléique réticulés neutralisées; un poly(acide 2-propénamide-co-2-propénoïque); et les mélanges et sels de ceux-ci.

14. Procédé selon la revendication 13, dans lequel les particules super-absorbantes sont faites d'un acide polyacrylique sodique ou du sel de sodium de poly(acide 2-propénamide-co-2-propénoïque).

15. Procédé selon la revendication 10, dans lequel le mélange comprend de 3 pour cent en poids à 5 pour cent en poids de particules super-absorbantes.

16. Embout de pipette qui comprend:
un tube creux ouvert à des première et seconde extrémités opposées;
un élément central disposé entre lesdites première et seconde extrémités opposées; et
un moyen pour attacher la première extrémité du tube creux à un dispositif d'aspiration;
dans lequel ledit élément central comprend le milieu auto-étanche poreux selon la revendication 1.

17. Pipette comprenant l'embout de pipette selon la revendication 16.

## Patentansprüche

1. Poröses selbstdichtendes Medium, das aufweist:
eine gesinterte poröse Kunststoffmatrix mit einer mittleren Porengröße; und
eine Vielzahl von Einschlüssen innerhalb der porösen Kunststoffmatrix, wobei die Einschlüsse aus einem superabsorbierenden Material bestehen;
wobei die mittlere Größe der Einschlüsse größer oder gleich der mittleren Porengröße ist.

2. Poröses selbstdichtendes Medium nach Anspruch 1, wobei die gesinterte poröse Kunststoffmatrix aus einem organischen Polymer besteht, das aus der Gruppe ausgewählt ist, die aus Acrylpolymeren; Polyolefinen; Polyestern; Polyamiden; Poly(ethersulfon); Polytetrafluorethylen; Polyvinylchlorid; Polycarbonaten, Polyurethanen und deren Gemischen besteht.

3. Poröses selbstdichtendes Medium nach Anspruch 2, wobei die gesinterte poröse Kunststoffmatrix aus Polyethylen besteht.

4. Poröses selbstdichtendes Medium nach Anspruch 1, wobei das superabsorbierende Material ein Polymer ist, das aus der Gruppe ausgewählt ist, die aus hydrolysiertem Stärke-Acrylnitril-Propfcopolymer; neutralisiertem Stärke-Acrylsäure-Propfcopolymer, verseiftem Acrylsäureester-Vinylaceteat-Copolymer; hydrolysiertem Acrylnitril-Copolymer; Acrylamid-Copolymer; modifiziertem vernetztem Polyvinylalkohol; neutralisierter selbstvenetzender Polyacrylsäure; vernetzten Polyacrylatsalzen; neutralisierten vernetzten Isobutylen-Maleinsäureanhydrid-Copolymeren; Poly(2-propenamid-co-2-propensäure) und deren Gemischen und Salzen besteht.

5. Poröses selbstdichtendes Medium nach Anspruch 1, wobei das superabsorbierende Material Natriumpolyacrylsäure oder das Natriumsalz von Poly(2-propenamid-co-2-propensäure) ist.

6. Poröses selbstdichtendes Medium nach Anspruch 1, wobei die Vielzahl von Einschlüssen in einem Anteil von 1 Gew.-% bis 30 Gew.-% der Matrix enthalten sind.

7. Poröses selbstdichtendes Medium nach Anspruch 1, wobei die Vielzahl von Einschlüssen in einem Anteil von 3 Gew.-% bis 5 Gew.-% der Matrix enthalten sind.

8. Poröses selbstdichtendes Medium nach Anspruch 1, wobei das selbstdichtende Medium einem Wassergegendruck von mehr als 0,48 bar (7 psi) widerstehen kann.

9. Poröses selbstdichtendes Medium nach Anspruch 1, wobei die mittlere Porengröße der porösen Kunststoffmatrix im Bereich von 15 µm bis 100 µm liegt.

10. Verfahren zur Herstellung eines porösen selbstdichtenden Mediums, wobei das Verfahren aufweist:
Ausbilden eines Gemischs aus:
Kunststoffteilchen und
superabsorbierenden Teilchen von einer mittleren Größe; und
Sintern des Gemischs bei einer Temperatur und einem Druck, die zur Bildung eines porösen Materials mit einer mittleren Porengröße ausreichen, die kleiner oder annähernd gleich der mittleren Größe der superabsorbierenden Teilchen ist.

11. Verfahren nach Anspruch 10, wobei die Kunststoffteilchen aus einem Polymer bestehen, das aus der Gruppe ausgewählt ist, die aus Acrylpolymeren; Polyolefinen; Polyestern; Polyamiden; Poly(ethersulfon); Polytetrafluorethylen; Polyvinylchlorid; Polycarbonaten, Polyurethanen und deren Gemischen besteht.

12. Verfahren nach Anspruch 10, wobei die Kunststoffteilchen aus Polyethylen bestehen.

13. Verfahren nach Anspruch 10, wobei die superabsorbierenden Teilchen aus einem Polymer bestehen, das aus der Gruppe ausgewählt ist, die aus hydrolysiertem Stärke-Acrylnitril-Propfcopolymer; neutralisiertem Stärke-Acrylsäure-Propfcopolymer, verseiftem Acrylsäureester-Vinylaceteat-Copolymer; hydrolysiertem Acrylnitril-Copolymer; Acrylamid-Copolymer; modifiziertem vernetztem Polyvinylalkohol; neutralisierter selbstvernetzender Polyacrylsäure; vernetzten Polyacrylatsalzen; neutralisierten vernetzten Isobutylen-Maleinsäureanhydrid-Copolymeren; Poly(2-propenamid-co-2-propensäure) und deren Gemischen und Salzen besteht.

14. Verfahren nach Anspruch 13, wobei die superabsorbierenden Teilchen aus Natriumpolyacrylsäure oder dem Natriumsalz von Natriumsalz von Poly(2-propenamid-co-2-propensäure) bestehen.

15. Verfahren nach Anspruch 10, wobei das Gemisch 3 Gew.-% bis 5 Gew.-% superabsorbierende Teilchen aufweist.

16. Pipettenspitze, die aufweist:
eine an gegenüberliegenden ersten und zweiten Enden offene Hohlröhre;
ein zwischen den gegenüberliegenden ersten und zweiten Enden angeordnetes Mittelglied; und
ein Mittel zum Befestigen des ersten Endes der Hohlröhre an einer Saugvorrichtung;
wobei das Mittelglied das poröse selbstdichtende Medium nach Anspruch 1 aufweist.

17. Pipette mit der Pipettenspitze nach Anspruch 16.
